(19) Europäisches Patentamt / European Patent Office / Office européen des brevets

(11) **EP 2 225 957 A1**

(12) **DEMANDE DE BREVET EUROPEEN**

(43) Date de publication:
08.09.2010 Bulletin 2010/36

(51) Int Cl.:
*A23L 1/30* (2006.01)          *A23L 1/03* (2006.01)
*A23L 1/20* (2006.01)

(21) Numéro de dépôt: **10159033.9**

(22) Date de dépôt: **24.04.1998**

(84) Etats contractants désignés:
**BE DE DK ES GB IT NL PT SE**

(30) Priorité: **24.04.1997  FR 9705067**

(62) Numéro(s) de document de la (des) demande(s)
initiale(s) en application de l'article 76 CBE:
**98922865.5 / 0 914 090**

(71) Demandeur: **Laboratoires Expanscience**
**92400 Courbevoie (FR)**

(72) Inventeurs:
• **Msika, Philippe**
**78000 Versailles (FR)**

• **Rancurel, Alain**
**28300 Leves (FR)**
• **Montaudoin, Marie-Georgette**
**28130 Maintenon (FR)**

(74) Mandataire: **Warcoin, Jacques et al**
**Cabinet Régimbeau**
**20, rue de Chazelles**
**75847 Paris Cedex 17 (FR)**

Remarques:
Cette demande a été déposée le 01-04-2010 comme
demande divisionnaire de la demande mentionnée
sous le code INID 62.

(54) **Composition anti-oxydante et/ou anti-élastase à base d'huile de lupin.**

(57)    La présente invention concerne une composition anti-oxydante et/ou anti-élastase contenant de l'huile de lupin ou une ou plusieurs fractions de celle-ci, son utilisation en cosmétologie, en pharmacie et en tant que complément alimentaire. Elle concerne plus particulièrement une composition comprenant un mélange d'huile de lupin et de concentrat d'huile de germe de blé, de préférence dans une proportion en poids de 70% d'huile de lupin et 30% de concentrat d'huile de germe de blé.

EP 2 225 957 A1

**Description**

La présente invention a pour objet de nouvelles compositions à base d'huile de lupin, ou de fractions de celle-ci, notamment des concentrats et des insaponifiables.

L'huile de lupin peut être extraite notamment à partir de farines et/ou de graines de lupin.

Le lupin est un proche parent du pois, de la fève, du soja et du haricot. La graine est traditionnellement employée en alimentation humaine pour sa forte teneur en protéines. Il est également incorporé dans l'alimentation des ruminants sous forme de la plante entière ou de ses graines et aussi fréquemment utilisé comme engrais vert. Plus particulièrement, quatre espèces de lupin présentent un réel intérêt agronomique : le lupin blanc (lupinus albus), le lupin bleu (lupinus angustifolius), le lupin jaune (lupinus luteus) et le lupin changeant (lupinus mutabilis).

Les végétaux constituent une source lipidique abondante et l'extraction d'huile végétale a déjà été largement réalisée. L'huile peut être alors utilisée directement, ou sous forme de certaines de ses fractions. Parmi les fractions susceptibles d'être obtenues à partir d'une huile végétale, on peut citer les insaponifiables ou encore les concentrats. Selon la Pharmacopée Européenne, 2ème édition, page V. 3.4.7 ; le terme « insaponifiable » s'applique aux substances, non volatiles, obtenues par extraction, avec un solvant organique d'une solution de la substance à examiner après saponification d'une huile végétale ou animale.

Par ailleurs, la méthode dite de distillation moléculaire permet de concentrer des huiles, issues notamment de plantes (huiles végétales) et d'obtenir des concentrats dans lesquels la concentration en insaponifiables peut atteindre par exemple de l'ordre de 10 % à 20 % en poids, voir même plus, cette concentration étant de l'ordre de 1% à 2% en poids dans les huiles de départ.

Des huiles végétales ont ainsi fait l'objet d'utilisations diverses, essentiellement en cosmétologie. Par exemple, le brevet FR 92 07830 décrit la préparation de compositions à base de fractions insaponifiables d'huiles de germe de blé et de sésame pour un usage cosmétique, ces concentrats étant obtenus par distillation moléculaire selon une méthode préférentielle décrite au brevet.

En ce qui concerne l'huile de lupin, aucune utilisation cosmétique ou pharmaceutique n'a été envisagée à ce jour. La publication de brevet européen EP-A-441672 décrit un procédé d'extraction de constituants d'une matière végétale permettant notamment d'obtenir une huile de lupin sans trace d'alcaloïde. Ce document décrit en particulier le traitement des graines de lupin amer et vise essentiellement à valoriser un tourteau protéique débarrassé de l'amertume caractéristique des graines de lupin amer. Les inventeurs ont maintenant constaté que, de façon surprenante, une composition contenant de l'huile de lupin ou une ou plusieurs fractions de celle-ci présentait un certain nombre de propriétés ouvrant la voie à des applications variées dans le domaine cosmétique, pharmaceutique ou alimentaire.

Les études à la base de invention ont permis de mettre en évidence que l'huile de lupin ou ses fractions exerce notamment une activité anti-oxydante et anti- élastase. La présente invention a donc pour objet une composition anti-oxydante et/ou anti-élastase contenant de l'huile de lupin ou une ou plusieurs fractions de celle-ci. L'huile de lupin peut être extraite à partir de farines et/ou de graines de lupin. L'huile de lupin peut être obtenue par toute méthode connue, en particulier par pression directe des graines de lupin.

On utilisera de préférence comme matière première pour l'obtention de l'huile de lupin ou de ses fractions, des espèces de lupin dites douces, c'est-à-dire dépourvues d'amertume. On peut citer notamment le lupin blanc, le lupin bleu, le lupin jaune et le lupin changeant en particulier le lupin blanc (lupinus albus). L'huile de lupin extraite de cette variété de lupin est alors dépourvue d'alcaloïdes indésirables.

L'invention vise plus particulièrement une composition comprenant de l'huile de lupin sous forme d'une fraction constituée par un concentrat d'huile de lupin obtenu par distillation moléculaire de ladite huile.

Selon un mode de réalisation préféré, les compositions à base d'huile de lupin selon l'invention comprennent une ou plusieurs fractions d'huile de lupin sous forme d'insaponifiable tel que contenu dans un concentrat d'huile de lupin obtenu par distillation moléculaire de ladite huile.

Avantageusement, la quantité en poids de la fraction insaponifiable dans le concentrat d'huile de lupin est de 30 % de 70 %, de préférence de 45 % à 65 % et de façon encore plus préférée, de l'ordre de 60 %.

Les inventeurs ont constaté que l'huile de lupin possède une teneur particulièrement élevée en dérivés polyphénoliques, ($\beta$-carotène et tocophérols et il est connu que les dérivés polyphénoliques contribuent à la stabilité à l'oxydation d'une composition les contenant. Avantageusement, l'invention a pour objet une composition anti-oxydante et/ou anti-élastase qui contient une fraction d'huile de lupin comprenant des dérivés phénoliques. D'une manière plus générale, l'invention concerne également toute composition anti-oxydante et/ou anti-élastase qui contient des dérivés phénoliques extraits de l'huile de lupin. De préférence, la teneur en dérivés phénoliques est au moins égale à 20 ppm.

L'invention a également pour objet une composition dans laquelle l'huile de lupin ou ses fractions sont en mélange avec de l'huile de germe de blé ou une ou plusieurs de ses fractions. Dans ce cas, la fraction d'huile de germe de blé utilisée peut être également un concentrat obtenu par distillation moléculaire de ladite huile ou encore une fraction insaponifiable contenue dans un tel concentrat. De façon surprenante, les inventeurs ont maintenant établi que l'huile de germe de blé ou ses fractions pouvait avantageusement être utilisée en combinaison avec l'huile de lupin ou ses fractions.

De préférence, lorsque les deux types d'huile sont présents dans une composition selon l'invention, l'huile de lupin est en mélange avec un concentrat d'huile de germe de blé.

Selon un mode de réalisation préféré de l'invention, les quantités en poids de concentrat d'huile de germe de blé et d'huile de lupin varient respectivement entre 10 % et 90 % et entre 90 % et 10 % de manière à ce que le total des quantités de ces deux huiles fasse 100 %.

De façon surprenante, on a par ailleurs constaté que dans un certain rapport de composition, l'activité anti-oxydante, en particulier antiradicalaire était nettement meilleure.

C'est pourquoi, une composition préférée selon l'invention, est celle dans laquelle les quantités en poids du concentrat d'huile de germe de blé et d'huile de lupin sont respectivement de 30 % et 70 %.

L'invention concerne également l'utilisation cosmétique d'une composition selon l'invention, notamment comme agent anti-oxydant, antiradicalaire, anti- élastase, protecteur UVA et/ou B, protecteur de l'ADN contre des dommages, notamment oxydatifs. Grâce aux activités mises en évidence pour les compositions selon l'invention, il est possible d'utiliser les compositions selon l'invention, à titre cosmétique ou pharmaceutique, notamment pour la photoprotection, contre le vieillissement actinique ou non, et pour la protection de la peau contre des agressions oxydantes y compris la pollution.

L'invention vise également les compositions selon l'invention, à titre de produit pharmaceutique, notamment dermatologique et plus particulièrement à titre d'agent destiné à la prévention ou au traitement des effets des UVA et/ou UVB sur la peau, aux niveaux épidermique, dermique, cellulaire ou extracellulaire, de produit pharmaceutique pour la prévention et le traitement des effets de l'oxydation, de l'élastase et des radicaux libres sur la peau, ou encore à titre d'agent ayant une activité de protection de l'ADN contre des dommages, notamment des dommages oxydatifs.

L'invention a également pour objet une méthode de traitement cosmétique comprenant l'application d'une composition anti-oxydante ou anti-élastase ou d'une composition cosmétique selon l'invention sur la surface cutanée d'un individu.

Sous un autre aspect, l'invention concerne une composition cosmétique ou pharmaceutique comprenant une composition selon l'invention, de préférence en association avec un véhicule physiologiquement acceptable.

Les compositions cosmétiques ou pharmaceutiques ainsi définies, sont susceptibles d'être utilisées notamment en tant que produit solaire protecteur des UVB et/ou A et/ou rayonnement infra-rouge, crème restructurante, raffermissante, produit, notamment crème pour la prévention et la régression des vergetures, crème nutritive, antiride (lutte contre le vieillissement de la peau épiderme et derme) et protectrice de jour, contour des lèvres et des yeux, sticks labiaux régénérants et protecteurs. Pour ces utilisations, les compositions cosmétiques selon l'invention sont avantageusement formulées pour un usage topique, notamment sous forme de crèmes, d'émulsions, de pommades, de sticks ou de gels.

Les compositions cosmétiques ou pharmaceutiques selon l'invention, peuvent présenter une teneur totale en poids en huile de lupin ou ses fractions et huile de germe de blé ou ses fractions de l'ordre de 0,5 à 10 % environ, de préférence d'environ 1% à environ 5%.

L'activité anti-oxydante des compositions selon l'invention, mise en évidence par la stabilité à l'oxydation notamment de l'huile de lupin et du concentrat correspondant est particulièrement avantageuse car elle ouvre des possibilités d'utilisations supplémentaires en tant que complément alimentaire mettant à profit cette activité anti oxydante.

Enfin, l'invention a également pour objet un procédé de préparation des compositions décrites ci-dessus. Différentes variantes sont envisageables selon les compositions. Ainsi, par exemple on peut citer :

- le mélange de deux concentrats d'huile de lupin et d'huile de germe de blé tels qu'obtenus par la méthode de distillation moléculaire par exemple telle que décrite dans la revue « Parfumerie Cosmétique et Arôme) » (1985, n° 61, page 91-96) ;
- le mélange préalable des huiles de lupin et de germe de blé suivi de la distillation moléculaire du mélange selon le procédé décrit ci-dessus ;
- le mélange d'huile de lupin et d'un concentrat d'huile de germe de blé obtenu par distillation moléculaire.

Le concentrat d'huile de germe de blé est avantageusement préparé selon le procédé de distillation moléculaire décrit au brevet FR 92 07830. Selon ce procédé, l'huile est étalée en couche mince sur la surface chauffée d'un rotor conique tournant à grande vitesse. On maintient un vide poussé dans l'enceinte de distillation. Dans ces conditions, il y a évaporation et non pas ébullition, depuis la surface chaude, des constituants de l'insaponifiable dont la séparation devient possible par rapport aux glycérides, l'avantage étant que l'huile et l'insaponifiable, réputés fragiles, ne sont pas dégradés au cours de l'opération.

L'invention sera davantage détaillée dans les exemples de réalisation qui suivent qui illustrent la préparation d'huile de lupin et de concentrat d'huile de germe de blé, séparément ou en mélanges, de formulations de compositions à base de tels mélanges et d'indications de leurs activités.

I. Exemples de préparation d'huile de lupin et d'insaponifiable d'huile de lupin EXEMPLE 1 : L'huile de lupin

On utilise des graines issues de semences certifiées (de lupinus albus), commercialisées par la société CANA.

Après un pré-nettoyage, les graines sont soigneusement nettoyées (élimination des graines et particules étrangères résiduelles, des graines cassées), et peuvent être décortiquées.

Elles sont aplaties dans un broyeur à cylindres ; après conditionnement hydrothermique à une température de 70°C environ, leur humidité varie entre 5% et 10%.

L'extraction de l'huile est alors réalisée dans un extracteur par percolation à l'hexane. L'extracteur étant rempli d'écailles broyées, l'extraction est réalisée par 4 à 6 lavages à l'hexane pour chaque charge.

Après chaque lavage, le miscella est pompé vers un distillateur ; après l'égouttage suivant le dernier lavage, le tourteau est envoyé vers un désolvanteur.

Le miscella est distillé en continu dans un distillateur chauffé par circulation de vapeur ; il est continuellement recyclé dans le distillateur.

A la fin des opérations, l'huile contenant encore de l'hexane est envoyée vers la distillation finale pour éliminer l'hexane sous vide (de 10mm à 35mm de mercure) entre 70°C et 100°C par « stripping » (strippage) pendant 10 mn.

La composition d'une huile brute extraite de graines selon le procédé ci- dessus est indiquée ci-après :

- Caractères organoleptiques : huile de couleur jaune orangé, d'odeur caractéristique
- Composition en acides gras :

  . Acide myristique C14      $\leq$ 0,50 %
  . Acide palmitique C16      4 à 10%.
  . Acide palmitoléique C16'      $\leq$ 2%
  . Acide stéarique C18      $\leq$ 4%
  . Acide oléique C18'      45 à 65 %
  . Acide linoléique C18"      9 à 17 %
  . Acide linolénique C 18'''      5 à 11 %
  . Acide arachidique C20      $\leq$ 3%
  . Acide gadoléique C20'      2 à 8%
  . Acide béhénique C22      $\leq$ 6%
  . Acide érucique C22'      $\leq$ 5%
  . Acide lignocérique C24      $\leq$ 2%

- Teneur en insaponifiable      $\geq$ 1,5g/100g
- Teneur en carotènes (en mg/100g)      $\geq$ 25mg/100g
- Teneur en tocophérols      $\geq$ 0,1g/100g
- Teneur en dérivés phénoliques (en équivalent      $\geq$ 20 ppm d'acide gallique)
- Teneur en alcool triterpénique (alpha-lupéol)      0,1 à 1%
- Teneur en stérols totaux :      $\geq$ 0,8g/100g

  . % relatif en campéstérol      18 à 24 %
  . % relatif en stigmastérol      5 à 10 %
  . % relatif en ß sitostérol      48 à 65 %
  . % relatif en delta 5-avenastérol      < 5%

EXEMPLE 2 : Préparation d'un concentrat d'huile de lupin par distillation moléculaire

La distillation moléculaire est réalisée par étalement de l'huile en couche mince sur la surface chauffée d'un rotor conique tournant à grande vitesse. On maintient un vide poussé dans l'enceinte de distillation. On introduit dans un appareil de distillation moléculaire approprié et de préférence du style centrifuge, de l'huile de lupin telle qu'obtenue à l'exemple 1. Le débit d'alimentation est de 10 à 30 kg par heure et de préférence entre 15 et 20 kg par heure.

Les paramètres de la distillation sont les suivants :

- température : 210°C à 250°C ;
- vide de là 10 $\mu$m (soit 0,13 à 1, 3 Pa).

Le pourcentage distillé étant proche de 10, on recueille soigneusement ce distillat.

La richesse en matière non saponifiable de cette fraction distillée est comprise entre 45 % et 65 %.

Les caractéristiques du concentrat ainsi obtenu sont les suivantes :

- Caractères organoleptiques : pâte jaune-orangé
- Teneur en squalène            ≈ 0,2g/100g
- Teneur en carotènes           ≈ 22,0mg/100g
- Teneur en tocophérols         ≈ 9,0g/100g

  . % relatif en alpha tocophérol      ≈1,0 %
  . % relatif en gamma tocophérol      ≈ 80,0 %
  . % relatif en delta tocophérol      ≈ 15,0 %

- Teneur en stérols totaux      ≈ 40,0g/100g

  . % relatif en campéstérol          ≈ 20,0 %
  . % relatif en stigmastérol         ≈ 9,0 %
  . % relatif en $\beta$ sitostérol          ≈ 60,0 %
  . % relatif en delta 5-avenastérol      ≈ 2,0 %
  . % relatif en delta 7-stigmastérol     ≈ 2,0 %

- Teneur en dérivés phénoliques, exprimée en acide gallique      ≈ 40,0ppm
- Teneur en insaponifiable total      ≈ 60,0 %

EXEMPLE 3 : Préparation de l'insaponifiable d'huile de lupin

Le concentrat d'huile de lupin obtenu à l'exemple 2 est saponifié dans un réacteur en acier inoxydable, dans les conditions suivantes :

On ajoute, pour 100 kg de concentrat, 20 kg d'hydroxyde de potassium en écailles, 250 kg d'alcool et 30 kg d'eau. On porte le mélange à reflux pendant 5 heures.

La solution hydroalcoolique des savons ainsi obtenue, est diluée de son volume avec de l'eau déminéralisée et extraite par du dichloroéthane (DCE) dans un appareil à contre-courant par exemple une colonne pulsée, qui extrait sélectivement la partie insaponifiable.

Cette solution d'insaponifiable est alors lavée par de l'eau dans un autre appareil à contre-courant de façon à éliminer les savons entraînés lors de l'extraction.

Le DCE est éliminé à raison de 95 % environ, sous pression atmosphérique dans un appareil d'évaporation à flot tombant, puis l'évaporation est terminée dans un appareil sous vide muni d'une double enveloppe et d'un injecteur permettant l'introduction de vapeur vive dans la masse, selon le mode opératoire suivant :

- Le produit est chauffé à 100°C, sous vide de 10mm de Hg (soit 1, 3 kPa) jusqu'à cessation de la distillation du dichloroéthane résiduel ; à ce moment, la vapeur d'eau est injectée dans la masse, à raison de 4% en poids d'eau par rapport au poids d'insaponifiable. La durée de l'opération est d'environ 4 heures.
- Le produit est séché par injection d'azote, en utilisant la tubulure ayant servi à l'introduction de la vapeur.
- Le produit est alors refroidi et le vide est cassé sous courant d'azote.

L'insaponifiable est conservé en fût polyéthylène haute densité et sac polyéthylène basse densité sous azote jusqu'à son utilisation.

L'insaponifiable d'huile de lupin ainsi obtenu est une pâte jaune orangé contenant :

- des tocophérols environ 3%

  . dont environ 96 % de gamma-tocophérol
  . et environ 4% d'alpha-tocophérol

- des stérols environ 40 % avec une teneur relative en :

  . campéstérol environ        25 %
  . stigmastérol environ       8%

. β sitostérol environ          52 %

EXEMPLE 4 : Mélange d'un concentrat d'huile de germe de blé et d'huile de lupin.

On prépare un concentrat d'huile de germe de blé selon le procédé décrit au brevet FR 92 07830, et on le mélange avec de l'huile de lupin telle qu'obtenue à l'exemple 1, dans des proportions respectives en poids de 30 % et 70 % par rapport au poids total du mélange. Les caractéristiques du mélange obtenu sont les suivantes :

- Caractères organoleptiques : huile limpide de couleur jaune orangé, d'odeur caractéristique.
- Composition en acides gras :

. Acide myristique C          14 2 %
. Acide palmitique C16        7 à 14 %
. Acide palmitoléique C16'     ≤ 2%
. Acide stéarique C18         ≤ 5%
. Acide oléique C18'          38 à 52 %
. Acide linoléique C18"       25 à 30 %
. Acide linolénique C18"'      4 à 11 %
. Acide arachidique C20       ≤ 3%
. Acide gadoléique C20'        2 à 8%
. Acide béhénique C22          1 à 6%
. Acide érucique C22'         ≤ 5%
. Acide lignocérique C24       ≤ 2%

- Teneur en insaponifiable          ≥ 4g/100g
- Teneur en carotènes (en mg/100g)        ≥15mg/100g
- Teneur en tocophérols          ≥ 500mg/100g
- Teneur en dérivés phénoliques          ≥ 14 mg/kg
- Teneur en stérols totaux :          ≥ 2,5 %

. % relatif en campéstérol          18 à 25 %
. % relatif en stigmastérol         3 à 10 %
. % relatif en β sitostérols        48 à 64 %
. % relatif en delta 5-avenastérol          ≤ 6%

II. Exemples de formulation (les produits désignés sous des appellations commerciales sont, sauf mention particulière, cités dans l'annuaire INCI ; 6ème édition.

1. <u>Crème à l'huile de lupin</u>

| | |
|---|---|
| Montanov 68 | 5,00% |
| Beurre de Karite | 3,00 % |
| Huile de paraffine | 10, 00 % |
| Cetearyl octanoate | 5, 00 % |
| Dimethicone | 1, 00 % |
| Huile de lupin | 5,00 % |
| Phénoxyéthanol | 0, 40 % |
| Phénonip | 0, 80 % |
| Eau | 59,10% |
| Glycérine | 10, 00 % |
| Controx VP | 0, 10 % |
| Parfun borealis n° 1 | 0,60 % |

2. <u>Crème hydratante légère à l'huile de lupin</u>

| | |
|---|---|
| Huile de vaseline épaisse | 2,00 % |
| Octyl dodecanol | 2,00% |
| Cetearyl glucoside | 5,00 % |
| Huile de jojoba | 1,00 % |
| Squalane | 1,00 % |
| Alcool cétostéarylique 25 OE | 1,00 % |
| ControxVP | 0,10% |
| Huile de lupin | 5,00 % |
| Eau purifiée | 72,475% |
| Citrate trisodique | 0,10 % |
| Acide citrique monohydrate | 0,025 % |
| SiliconeQ21401 * | 4,00% |
| Conservateur GD 7000** | 0, 20 % |
| Sepigel 305 | 0,80% |
| Aloe vera gel | 5, 00 % |
| Parfum composition 53905-1 Synarome | 0,30 % |

\* Le silicone Q21401 est commercialisé par la société Dow Corning sous la désignation Cyclométhicone et diméthiconol ;

\*\* Le conservateur GD 700 est commercialisé par la société PHYTOCOS sous : propylène glycol, eau, phénoxyéthanol, méthylparaben, butylparaben, isobutylparaben, éthylparaben ; méthylchlorothiazolinone et méthylisothiazolinone

3. <u>Crème au concentrat d'huile de lupin</u>

| | |
|---|---|
| Esters d'acides gras polyoxyéthylénés | 4,00 % |
| Acide stéarique | 2,00 % |
| Alcool cétylique | 2,00 % |
| Huile de vaseline fluide | 2,00 % |
| Propylène glycol | 2,00 % |
| Glycérine | 1,50 % |
| Concentrat d'huile de lupin | 1,00 % |
| Polyimidazole urée | 0,20 % |
| Parahydroxybenzoate de méthyle | 0,20 % |
| Parahydroxybenzoate de propyle | 0,10 % |
| Acide tartrique | 0,02 % |
| Parfum | 0,30 % |
| Eau | qsp100,00 % |

4. <u>Crème à l'insaponifiable de lupin</u>

| | |
|---|---|
| Monostearate de sorbitan polyoxyéthyléné | 3,20 % |
| Octyldodecanol | 3,00 % |

(suite)

| | |
|---|---|
| Huile d'amande douce | 2,80 % |
| Monostéarate de sorbitan | 2,00 % |
| Huile de vaseline fluide | 2,00 % |
| Laurate d'hexyle | 2,00 % |
| Cire d'abeille | 2,00 % |
| Alcool cétylique | 1,50 % |
| Stéarate de diéthylène glycol | 1,50 % |
| Mélange de monoglycérides d'alcools gras de triglycérides et d'esters cireux | 1,00 % |
| Insaponifiable de lupin | 1,00 % |
| Parahydroxybenzoate de méhyle | 0,30 % |
| Parahydroxybenzoate de propyle | 0,10 % |
| Parfum | 0,25 % |
| Eau | qsp 100,00 % |

5. Crème anti-âge avec un mélange comprenant 30 % de concentrat d'huile de germe de blé et 70 % d'huile de lupin

| | |
|---|---|
| Arlacel 165 | 5,00 % |
| Cetearyl octanoate | 14,00 % |
| Plamitate de cetyle | 1,00% |
| Concentrat d'huile de germe de blé et huile de lupin | 3,00 % |
| Phenonip | 0,80 % |
| Phenoxyéthanol | 0,40 % |
| Controx VP | 0,10 % |
| Eau | 72,50 % |
| Sepigel | 2,50 % |
| Parfum Firmenich petit matin | 0,70 % |

6. Lait après-soleil avec un mélange comprenant 30 % d'un concentrat d'huile de germe de blé et 70 % d'huile de lupin

| | |
|---|---|
| Emulgade SE* | 6,00 % |
| Miglyol 812 | 3,00 % |
| Cetearyl Octanoate | 3,00 % |
| Beurre de Karite | 2,00 % |
| Alcool cetylique | 1,00 % |
| Concentrat d'huile de germe de blé et huile de lupin | 1,50 % |
| Silicone Q2 1401 | 2, 00 % |
| Eau | 76,90 % |
| Glycérine | 3, 00 % |
| Controx VP | 0, 10 % |
| Phenonip | 0, 80 % |
| Phenoxyéthanol | 0,40 % |
| Parfum eau marine TM 4509 Technicolor | 0, 30 % |
| * Emulgade SE est commercialisé par la société Henkel sous : Glyceryl stearate, ceteareth-20, ceteareth-12, cetearyl alcohool et cétyl palmitate. | |

7. <u>Crème solaire avec un mélange comprenant 30 % d'un concentrat d'huile de germe de blé et 70 % d'huile de lupin</u>

| | |
|---|---|
| Eau | qsp 100 % |
| Caprylic/Capric Triglyceride | 16,10% |
| Dioxyde de titane | 10,00 % |
| Octyl Palmitate | 4,64 % |
| Benzoate d'alkyle C12-15 | 4,00 % |
| Cyclomethicone | 3,00 % |
| Cetearyl Octanoate | 3,50% |
| Cetyl Dimethicone Copolyol | 2,50 % |
| Oxyde de zinc | 2,00 % |
| Aluminium/Magnesium Hydroxide Stearate | 2, 00 % |
| Cetyl Dimethicone | 1,00 % |
| Acide isostéarique | 1,00 % |
| Chlorure de sodium | 1,00 % |
| Phenoxyethanol | 0,876 % |
| Tocopheryl acetate | 0,50 % |
| Octyldodecanol | 0,28725 % |
| Merthylparaben | 0,128% |
| Butylparaben | 0,048 % |
| Concentrat d'huile de germe de blé et huile de lupin | 2,00 % |

8. <u>Crème solaire avec un mélange comprenant 30 % d'un concentrat d'huile de germe de blé et 70 % d'huile de lupin</u>

| | |
|---|---|
| Eau | qsp 100 % |
| Octyl Methoxycinnamate | 7,50 % |
| Octyl Cocoate | 10,00 % |
| Octyl Salicylate | 5,00 % |
| Oleth-2 | 3,00 % |
| Benzophenone-3 | 3,00 % |
| Huile de vaseline | 1,80 % |
| Stearyl Heptanoate | 1,425 % |
| Stearamine Oxide | 1, 30 % |
| Acrylates/Octylacrylamide Copolymer | 1,30 % |
| Sulfate de Magnesium | 0,50 % |
| Vitamine E | 0,50 % |
| Sodium Magnesium Silicate | 0,40 % |
| Phenoxyethanol | 0,053 % |
| Methylparaben | 0,012 % |
| Parfum | 0,20 % |
| Concentrat d'huile de germe de blé et d'huile de lupin | 1,00 % |

III. Etude de l'activité antiradicalaire de mélange d'huile de lupin et du concentrat d'huile de germe de blé : comparaison avec la vitamine E et la vitamine C associée au glutathion.

1. <u>Produits testés</u>

Cinq échantillons référencés ci-après ont été testés :

- L0 : huile de germe de blé
- L50 : mélange de 50 % de concentrat d'huile de germe de blé et 50 % d'huile de lupin
- L70 : mélange de 30 % de concentrat d'huile de germe de blé et 70 % d'huile de lupin
- L90 : mélange de 10 % de concentrat d'huile de germe de blé et 90 % d'huile de lupin
- L 100 : huile de lupin pure

L'activité de ces cinq lots a été comparée à celle de l'acétate de vitamine E (Sigma), d'une association ascorbate de sodium (vitamine C) et glutathion (Sigma), ainsi qu'à celle de la ($\beta$-carotène à 30 % en solution dans un glycéride synthétique (Référence 1) et à celle d'un mélange de 50 % en poids de concentrat d'huile de germe de blé et 50 % en poids de concentrat d'huile de sésame (tel que décrit au brevet FR 92 07830) (Référence 2).

2. <u>Matériels et méthodes</u>

2.1. Cultures cellulaires

Les cellules sont des kératinocytes épidermiques humains. On s'est procuré des kératinocytes à partir d'un déchet opératoire résultant d'une intervention de plastie mammaire réalisée chez une femme âgée de 40 ans (donneur BIOPREDIC n° VACHLOOO9). Les cellules sont utilisées au sixième passage, elles sont ensemencées à 1 x 105 cellules par puits dans des plaques de culture de 6 puits. Elles sont utilisées à confluence.

2.2. Evaluation de l'activité antiradicalaire

Elle est quantifiée par une méthode fluorométrique qui met en évidence le taux d'hydroperoxydes qui sont des métabolites des radicaux libres. On utilise une sonde appropriée qui se transforme en un dérivé fluorescent en leur présence, selon la méthode décrite par Keston A. S. and Brandt R. (The fluorometric analysis of ultramicro quantities ofhydrogenperoxide, Anal. Biochem. 11,1965,1-5).

2.3. Irradiation aux U.V.A. et observations des effets de l'irradiation

Les radicaux libres sont générés au niveau de la culture cellulaire par irradiation par les rayonnements ultraviolets A. Les kératinocytes sont irradiés par des rayons UVA du côté inférieur de la monocouche (pour éviter les éventuels effets filtres des produits à l'essai) à l'aide d'une table à ultraviolets (VILBERT LOURMAT) pendant 90 minutes (10 J/cm2) Les produits à l'essai ainsi que les deux produits de référence, référence 1 et référence 2, sont solubilisés dans du diméthylformamide (DMF).

Les produits à l'essai, L0, L50, L70, L90 et L100 sont testés à des concentrations de 0,000001 ; 0,00001 ; 0,0001 ; 0,001 et 0,01 % (p/v) (soit 1 ; 10 ; 100 ; 1000 et 10000 ppm) dans le tampon HBSS (solution saline de Hacks) contenant 0,04 % (v/v) de DMF.

La concentration en DMF est maintenue constante à 0,04 % (v/v) dans chaque dilution.

Les références 1 et 2 sont testées à 0,01 % (p/v) dans le tampon HBSS contenant 0,04 % (v/v) de DMF Le mélange vitamine C à 0,5 mg/ml + glutathion à 0,5 mg/ml et l'acétate de vitamine E à 0,1 % (p/v) sont directement solubilisés dans le tampon HBSS.

Le mélange vitamine C à 0,5 mg/ml + glutathion à 0,5 mg/ml et l'acétate de vitamine E à 0,1% (p/v) sont directement solubilisés dans le tampon HBSS.

La sonde destinée à mettre en évidence les hydroperoxydes (sonde CM-H2DCF-DA commercialisée par MOLECULAR PROBES) a été utilisée à 5$\mu$M dans le milieu d'essai.

Les kératinocytes sont incubés à températures ambiante avec les produits à l'essai et les produits de référence pendant l'irradiation, soit 90 minutes.

2.4. Témoins

Des kératinocytes témoins irradiés (+UVA) ou non irradiés (-UVA) par des rayons LTVA sont incubés dans le tampon HBSS (avec témoin (DMF) ou sans (DMF) à 0,04 % (v/v)) et contenant seulement la sonde pendant la durée de l'irradiation.

Après irradiation, les kératinocytes sont lysées par action des ultrasons. Les lysats cellulaires sont transférés dans des plaques de 96 puits et analysés par fluorimétrie à l'aide d'un analyseur de plaques (excitation : 355 nm, émission : 460 nm).

Les résultats sont exprimés en unité de fluorescence/puits de culture

3. <u>Résultats</u>

Les groupes de données (groupe de témoin et groupes traités) ont été comparés par une analyse de la variance à un facteur (ANOVA1), suivie par un test de Dunnett.

Le pourcentage de protection des produits à l'essai ou des produits de référence R1 et R2 a été calculé à l'aide de la formule suivante :

$$\% \text{ de protection} = \frac{\left[\begin{array}{l}\text{Valeur moyenne obtenu}\\\text{avec le témoin irradié}\end{array}\right] - \left[\begin{array}{l}\text{Valeur moyenne obtenu}\\\text{avec les produits à l'essai}\\\text{ou les produits de référence}\end{array}\right]}{\left[\begin{array}{l}\text{Valeur moyenne obtenu}\\\text{avec le témoin irradié}\end{array}\right] - \left[\begin{array}{l}\text{Valeur moyenne obtenu}\\\text{avec le témoin non irradié}\end{array}\right]} \times 100$$

Par convention, lorsque les valeurs mesurées en présence des produits à l'essai ou en présence des produits de référence étaient inférieures à celles du « témoin non irradié », le pourcentage de protection a été rapporté à 100 %. De même, lorsque ces valeurs étaient supérieures à celles du « témoin irradié », le pourcentage de protection a été rapporté à 0%.

Les tableaux ci-après (Tableaux 1 à 7) présentent les résultats obtenus exprimés en % de protection.

Tableau 1

|  | témoin -UVA | témoin + UVA | vitamine C+ glutathion | acétate de vitamine E | réf 1 0,01% (p/v) | réf 2 0,01% (p/v) |
|---|---|---|---|---|---|---|
| % de protection | 100 | 0 | 100 | 65 | 62 | 93 |

Tableau 2

|  | Témoin DMF -UVA | Témoin DMF + UVA | L0 (% p/v) | | | | |
|---|---|---|---|---|---|---|---|
|  |  |  | 0,000001 | 0,00001 | 0,0001 | 0,001 | 0,01 |
| % protection | 100 | 0 | 64 | 54 | 96 | 28 | 23 |

Tableau 3

|  | Témoin DMF -UVA | Témoin DMF + UVA | L50 (% p/v) | | | | |
|---|---|---|---|---|---|---|---|
|  |  |  | 0,000001 | 0,00001 | 0,0001 | 0,001 | 0,01 |
| % protection | 100 | 0 | 38 | 60 | 66 | 17 | 58 |

Tableau 4

|  | Témoin DMF -UVA | Témoin DMF + UVA | L70 (% p/v) | | | | |
|---|---|---|---|---|---|---|---|
|  |  |  | 0,000001 | 0,00001 | 0,0001 | 0,001 | 0,01 |
| % protection | 100 | 0 | 51 | 95 | 86 | 0 | 0 |

Tableau 5

| | Témoin DMF -UVA | Témoin DMF + UVA | L90 (% p/v) | | | | |
|---|---|---|---|---|---|---|---|
| | | | 0,000001 | 0,00001 | 0,0001 | 0,001 | 0,01 |
| % protection | 100 | 0 | 8 | 64 | 56 | 60 | 27 |

Tableau 6

| | Témoin DMF -UVA | Témoin DMF + UVA | L100 (% p/v) | | | | |
|---|---|---|---|---|---|---|---|
| | | | 0,000001 | 0,00001 | 0,0001 | 0,001 | 0,01 |
| % protection | 100 | 0 | 24 | 26 | 33 | 79 | 87 |

Les résultats reportés aux Tableaux 1 à 6 permettent de tirer les conclusions suivantes :

- Le DMF à 0,04 % (v/v) utilisé comme solvant intermédiaire des produits à tester et des produits de référence (réf. 1 et réf. 2) n'a pas d'effet significatif vis à vis de l'intensité de la fluorescence émise par la sonde, avant et après irradiation des cellules.
- Les deux produits de référence vitamine C + glutathion d'une part et acétate de vitamine E d'autre part, présentent un effet protecteur attendu de 100 % et 65 % respectivement vis à vis de l'effet délétère des rayons UVA.
- Les produits de référence, réf. 1 et réf. 2 testés à 0,01 % (p/v) présentent un effet protecteur de 62 % et 93 % respectivement vis à vis de l'effet délétère des rayons UVA.

En ce qui concerne les produits à tester, on observe les résultats suivants :

- un effet protecteur est obtenu avec les produits L 0, L 50, L 70, L 90 et L 100 vis à vis des effets délétères des rayons UV riches en UVA.
- le produit L 0 (100 % de concentrat d'huile de germe de blé) est plus protecteur aux faibles concentrations testées qu'aux fortes concentrations.
- un effet inverse est observé pour le produit L 100 (100 % d'huile de lupin).
- les mélanges L 50 et L 90 ne sont pas meilleurs que les produits L 0 et L 100.
- par contre, de façon inattendue, le produit L 70, notamment à une concentration de 0,0001 % (p/v) est significativement plus protecteur que les quatre autres produits.

IV. Etude de l'activité anti élastase du produit L 70 dans un modèle d'explant de peau humaine.

L'étude de l'activité anti élastase représente un bon indice du vieillissement de la peau puisqu'il est maintenant bien connu que la dégradation des fibres élastiques présentes dans le derme met en jeu des élastases endogènes. Le test utilisé permet d'étudier l'activité anti élastase in vitro dans des coupes d'explant de peau humaine. Une application d'élastase purifiée sur une partie limitée de la coupe s'accompagne d'une dégradation des fibres élastiques endogènes. Les fibres élastiques sont colorées par la (+) catéchine. Le pourcentage de fibres élastiques intactes par champ optique est évalué par analyse d'images. Un produit présentant une activité anti élastase permet de conserver l'intégrité des fibres élastiques en présence d'élastase purifiée.

1. Produits testés

Le produit L 70 testé précédemment pour son activité anti radicalaire a été comparé à des produits anti élastase de référence : l'élastinal (Sigma) et le chlorure de mercure (Sigma).

2. Matériels et Méthodes

2.1. Réactifs

On utilise une élastase pancréatique (type IV, référence E0258, Sigma). Le milieu d'incubation des coupes de peau humaine ( « véhicule ») est le tampon Hépés 0,1 M, ajusté à pH 7.5 avec la soude et contenant 0,1 M de chlorure de sodium.

2.2. Système d'essai

Les coupes de peau sont réalisées à partir d'un déchet opératoire recueilli après une plastie abdominale. Le donneur était une femme âgée de 37 ans. On réalise des explants de 1 cm de diamètre et on les dépose sur un support de liège et on les congèle à-80°C. On réalise des coupes transverses de 6 μm d'épaisseur avec un cryomicrotome. Les coupes sont fixées sur des lames de verre et maintenues hydratées avec le véhicule pendant l'essai.

2.3. Préparation des produits à tester et des produits de référence, incubation avec le système d'essai

Les produits à tester, le sont à 0,5 ; 1 et 5% (v/v) dans le véhicule.
L'élastatinal est testé à 0,01 et 0,1% (p/v) dans le véhicule.
Le chlorure de mercure est testé à 0,025 et 0,125% (p/v) dans le véhicule.
Les dilutions des produits à tester et des produits de référence sont déposées sur les coupes de peau, à raison de 100 μl par coupe (0,32 cm$^2$ de surface), et pré-incubées pendant dix minutes à 37°C. Des bandes de papier filtre (0,16 cm$^2$ de surface) imbibées de véhicule seul (témoin véhicule) ou contenant de l'élastase pancréatique à 5 unités internationales (UI)/ml (témoin enzyme) sont déposées sur les coupes. Les lames sont placées en chambre humide à 37°C pendant trois heures.

2.4. Evaluation de l'activité anti élastase

Après l'incubation, les coupes sont rincées avec le milieu d'incubation et colorées par la (+) catéchine. L'activité de l'enzyme en absence et en présence des produits à tester ou des produits de référence est évaluée par analyse d'image : l'image des coupes colorées est digitalisée sur un écran vidéo ; un logiciel d'analyse d'image permet de mesurer les niveaux de gris des images binarisées ; on mesure la surface occupée par les fibres élastiques intactes. Les résultats sont exprimés en pourcentage de fibres élastiques intactes par champ optique.
Le pourcentage d'inhibition de l'activité élastase des produits à l'essai et des produits de référence est calculé à l'aide de la formule suivante :

$$\% \text{ d'inhibition} = \dfrac{\left[ \begin{array}{c} \text{Valeur obtenue} \\ \text{avec le témoin enzyme} \end{array} \right] - \left[ \begin{array}{c} \text{Valeur obtenue avec} \\ \text{avec les produits à l'essai} \\ \text{ou les produits de référence} \end{array} \right]}{\left[ \begin{array}{c} \text{Valeur obtenue} \\ \text{avec le témoin véhicule} \end{array} \right] \qquad \left[ \begin{array}{c} \text{Valeur obtenue} \\ \text{avec le témoin véhicule} \end{array} \right]} \times 100$$

Les résultats sont montrés au tableau ci-après (Tableau 7)

Tableau 7

| Produit | Concentration % (v/v) | % d'inhibition |
|---|---|---|
| Témoin véhicule | - | 100 |
| Témoin enzyme | - | 0 |
| Chlorure de mercure | 0,025 (p/v) | 18,4 |
| | 0,125 (p/v) | 39,3 |
| Elastatinal | 0,01 (p/v) | 71,5 |
| | 0,1 (p/v) | 59,9 |

(suite)

| Produit | Concentration % (v/v) | % d'inhibition |
|---------|----------------------|----------------|
| L 70 | 0,5 | 31 |
| | 1 | 70 |
| | 5 | 70 |

Le calcul du pourcentage d'inhibition de l'activité élastase donne de façon attendue 100 % en absence d'élastase (témoin véhicule) et 0% en présence d'élastase (témoin enzyme).

L'élastatinal, testé à 0,01 et 0,1 % (p/v) inhibe respectivement de 71 % et 60 % l'activité de l'élastase. Les fibres d'élastine sont en grande partie intactes dans la zone d'application de l'élastase.

Le chlorure de mercure, testé à 0,025 et 0,125 % (v/v), inhibe respectivement de 18 % et 39 % la dégradation des fibres d'élastine par l'élastase.

L 70, testé à 0,5 ; 1 et 5% (v/v), inhibe respectivement de 31 %, 70 % et 70 % la dégradation des fibres d'élastine par l'élastase.

V. Etude de l'activité anti-élastase d'une huile de Lupin brute telle qu'obtenue à l'exemple 1 et d'une huile de Lupin obtenue par pression directe des graines de Lupin (huile de lupin pression).

L'huile de lupin pression est obtenue par pression à froid, avec une presse de type titan, des graines de lupin de l'espèce Lupinus albus décortiquées. Le tourteau est recyclé en continu afin d'extraire l'huile résiduelle. Après l'étape de pression, l'huile est stockée à température ambiante dans une cuve appropriée pendant 24 heures.

Elle est ensuite filtrée afin d'éliminer la matière solide en suspension telle que fibres ou phopholipides.

Les caractéristiques de l'huile de lupin pression obtenue sont les suivantes :

- Caractères organoleptiques : huile de couleur jaune orangé, d'odeur caractéristique.

- Composition en acides gras :

| | |
|---|---|
| acide myristique C14 | $\leq$ 0,50 % |
| acide palmitique C16 | 4 à10% |
| acide palmitoléique C16' | $\leq$ 2% |
| acide stéarique C18 | $\leq$ 4% |
| acide oléique C18' | 45 à 65 % |
| acide linoléique C18" | 9 à 17 % |
| acide lino lénique C 18''' | 5 à 11 % |
| acide arachidique C20 | $\leq$ 3 % |
| acide gadoléique C20' | 2 à 8 % |
| acide béhénique C22' | $\leq$ 6 % |
| acide érucique C22 | $\leq$ 5% |
| acide lignocérique C24 | $\leq$ 2 % |

- Teneur en insaponifiable          $\leq$ 1,5 g/100 g
- Teneur en carotènes (en mg/100 g)          environ 25 mg/100 g
- Teneur en tocophérols          environ 120 mg/100 g
- Teneur en dérivés phénoliques
(en équivalent d'acide gallique)          environ 30 ppm
- Teneur en alcool triterpénique (alpha-lupéol) 0, 1 g à 1 %
- Teneur en stérols totaux $\leq$ 0,8 g/100 g

| | |
|---|---|
| % relatif en campéstérol | 18 à 24 % |
| % relatif en stigmastérol | 5 à 10 % |
| % relatif en $\beta$ sitostérol | 48 à 65 % |
| % relatif en delta 5-avenastérol | < 5 % |

L'activité anti-élastase est déterminée dans les conditions indiquées au IV ci- dessus. Les résultats obtenus sont rassemblés dans le tableau ci-après.

Tableau 8

| L'élastatinal et le chlorure de mercure ont été utilisés comme produit de référence. | | |
|---|---|---|
| Produit | Concentration % (v/v) | Inhibition |
| Témoin véhicule | - | +++ |
| Témoin enzyme | - | 0 |
| Chlorure de mercure | 0,125 (p/v) | ++ |
| Elastatinal | 0,01 (p/v) | ++ |
| Huile de lupin brute | 0,5<br>1<br>5 | +<br>++<br>++ |
| Huile de lupin pression | 0,5<br>1<br>5 | +<br>+++<br>+++ |

Comme attendu, en absence d'élastase (témoin véhicule), on n'observe aucune dégradation des fibroblastes.

Comme attendu, l'élastatinal, testé à 0,1 % (p/v), et le chlorure de mercure, testé à 0,125 % (p/v), inhibent fortement l'activité enzymatique de l'elastase. L'activité anti-élastase de l'huile de lupin brute et celle de l'huile de lupin pression augmentent avec la concentration. A une concentration de 5 % (v/v), l'huile de lupin pression inhibe totalement l'activité enzymatique de l'élastase.

VI. Etude de l'activité anti oxydante d'une composition à base d'huile de lupin ou de ses fractions dans le test au Rancimat.

L'activité anti oxydante est évaluée par le test au Rancimat. Ce test représente une adaptation du test de Swift pour être automatisé sur un appareil Rancimat (une version du test au Rancimat est commercialisée par la Société METROHM, Suisse).

Le tableau ci-après (Tableau 9) indique les résultats de stabilité au Rancimat 98°C, 201/h) pour des compositions selon l'invention et, à titre de comparaison, pour d'autres huiles d'origine végétale.

Tableau 9

| ECHANTILLONS ANALYSES | RESULTATS |
|---|---|
| Huile de lupin | 60,4 |
| Huile de tournesol | environ 10 h |
| Huile de maïs | 18,2 h |
| Huile de sésame | 20,7 |
| Concentrat d'huile de lupin | > 75 h |

VIII. Etude de l'activité antioxydante de fractions d'huile de Lupin par une méthode de vieillissement accéléré.

Les résultats sont reportés dans le tableau 10 ci-après.

Tableau 10

| Produits | Concentration dans l'huile (% poids) | Indice de peroxyde de départ (m équivalent/kg) | Indice de peroxyde après 13 jours (m équivalent/kg) |
|---|---|---|---|
| Huile végétale dénaturée | - | 3,4 | 18,5 |
| Concentrat | 2,5 | 6,4 | 10,1 |
| Insaponifiable | 2,3 | 2,8 | 6,7 |

Le témoin utilisé est une huile végétale dénaturée dont on a extrait par distillation moléculaire la quasi totalité de l'insaponifiable. Elle contient essentiellement des triglycérides qui sont des substrats facilement oxydables.

Le concentrat d'huile de lupin est tel qu'obtenu à l'exemple 2 et l'insaponifiable est tel qu'obtenu à l'exemple 3.

Après mise en solution dans l'huile dénaturée, l'activité anti-oxydante des fractions est testée par une méthode de vieillissement accéléré. Un film mince d'huile, de 2 à 3 mm d'épaisseur, est exposé à l'air pendant 13 jours, à 50°C.

La dégradation oxydative est évaluée par mesure de l'indice de peroxyde.

L'effet anti-oxydant des différentes fractions est d'autant plus marqué que l'indice de peroxyde après 13 jours d'exposition est faible.

On constate que la résistance à l'oxydation du témoin est augmenté en présence de concentrat d'huile de lupin et encore davantage en présence d'insaponifiable. Ces résultats démontrent donc l'activité antioxydante de ces fractions d'huile de lupin.

IX. Etude de l'effet protecteur de l'ADN.

On a également observé que les compositions selon l'invention avaient un effet protecteur sur l'ADN testé en utilisant un système générateur d'espèces oxygénées réactives induisant la formation de lésions sur l'ADN. Ce test a permis de mettre en évidence, en particulier pour la composition à base de 30 % de concentrat d'huile de germe de blé et de 70 % d'huile de lupin (L70), une inhibition significative des dommages créés par une espèce oxygénée réactive (ROS), l'oxygène singulet $O_2$ généré par éclaircissement du bleu de méthylène, révélatrice de l'effet protecteur.

1. <u>Produits testés</u>

Les produits de l'invention L70, L100, sont comparés à l'huile de lupin raffinée et à un témoin (ß carotène à 3mg/100g).

2. <u>Principe du test utilisé</u>

Le test utilisé est un système biologique de détection de dommages (essai 3- D) sur de l'ADN capté sur microplaque (Analytical Biochemistry, 1995, 232, 37-42). Les dommages sont reconnus par les enzymes de réparation et réparés par des extraits cellulaires purifiés. La réparation des lésions implique une phase d'excision puis de resynthèse du fragment d'ADN endommagé et excisé. Au cours de l'étape de synthèse réparatrice, un (ou des) nucléotide (s) modifié (s) (dUTP digoxigénylé ou DIG-11-dUTP) est (sont) incorporé (s) dans l'ADN. Ces nucléotides sont ensuite reconnus par anticorps anti-DIG couplé à la phosphatase alcaline. Un substrat de la phosphatase alcaline (Lumi-Phos 530) est ensuite ajouté et le signal luminescent émis est mesuré par un luminomètre (Lumax 2, commercialisé par la société S. F. R. 1.). L'intensité du signal recueilli est fonction de différents paramètres (extraits, concentration en sels, quantité d'ADN, temps de réaction...) dont celui relatif au nombre de lésions réparées, donc de lésions présentes sur l'ADN. Une relation dose-réponse est observée dans la limite de 1 à 15 lésions pour 6 kilobases pour la plupart des lésions.

Ce système est susceptible de réparer tout type de lésion puisque toutes les enzymes de réparation des lésions de l'ADN sont présentes et actives dans les extraits. Les dommages oxydatifs sont donc reconnus dans ce système.

Les dommages sont créés par des espèces oxygénées réactives (ROS) au moyen d'un système qui induit la formation de lésions sur l'ADN adsorbé dans un puits, conduisant à la lecture d'un signal de synthèse réparatrice. En présence d'un composé ou d'un mélange de composés permettant de protéger l'ADN, du fait par exemple de propriétés antioxydantes ou antiradicalaires, on observe une diminution, voire une abolition du signal de réparation, reflet de la quantité de lésions.

On génère, par éclaircissement du bleu de méthylène, le ROS $^1O_2$, puissant électrophile qui possède une durée de vie très courte. Il réagit donc très rapidement avec des bases de l'ADN et produit des dommages divers de type modification de bases ou perte de bases, très génotoxiques.

3. <u>Matériels et Méthodes</u> 3.1. Matériel

Les réactifs utilisés sont décrits dans l'article Analytical Biochemistry, 1995, 232,37-42.

Le signal chimioluminescent est détecté à l'aide d'un luminomètre lumax 2 commercialisé par la société S. F. R. 1.

3.2. Méthodes

3.2.1. Adsorption de l'ADN cible dans les puits de microplaque

On met en contact de l'ADN plasmidique (pBS) ultrapurifié (forme superenroulée majoritaire) avec les puits sensibilisés par de la poly-lysine pendant 30 minutes à 30°C sous agitation légère à une concentration de 1μg/ml dans

un volume de 50μl. Dans ces conditions, l'adsorption de l'AND est quantitative (environ 40 ng par puits).

On ajoute un contrôle positif de réparation consistant en un plasmide préendommagé aux UV-C (appelé pBS^UV).

Après l'incubation, les puits sont lavés 2 fois avec une solution de PBS additionnée de Tween 20 à 0,1 %.

3.2.2. Génération de ROS par éclairement de bleu de méthylène

Une solution stock de bleu de méthylène à 10 μg/ml est diluée à une concentration de 4 ng/ml dans de l'eau ultrapure (qualité MilliQ de Millipore).

Cette solution est mélangée à volume égal avec différentes dilutions des échantillons, et 50 μl du mélange (à 2ng/ml de bleu de méthylène) sont ajoutés dans les puits contenant l'ADN plasmidique adsorbé. Les puits sont posés sur la glace et éclairés 20 minutes par 2 lampes de 100W, distantes de 30 cm.

3.2. 3. Dilutions des échantillons à tester dans le test de protection de l'ADN

Les quatre échantillons ont été testés à 3 dilutions. Les résultats sont présentés au Tableau 9.

Les dilutions ont été réalisées 2X, dans du propylène glycol, afin d'obtenir les dilutions voulues après addition d'un volume de solution de bleu de méthylène de 4 ng/ml.

Afin de vérifier que l'abaissement du signal de réparation n'est pas aspécifique, les mêmes dilutions (1X) sont incubées dans les mêmes conditions sur du pBS^UV. Une dilution ne présentera un effet protecteur que si l'on note un abaissement du signal de réparation, à cette dilution, en présence de bleu de méthylène, et une absence de modification du signal sur le pBS^UV. Une inhibition du signal de réparation contrôle du pBS^UV peut par exemple être expliquée par une interaction directe de l'échantillon avec l'ADN qui bloque l'accès des lésions aux enzymes de réparation.

Ces différentes dilutions ont été incubées sur de l'ADN non endommagé (pBS) et éclairées en l'absence de bleu de méthylène. Ce test permet de vérifier que le produit testé n'est pas génotoxique.

Tableau 11

| Composé testé | Dilution ou concentration | % d'inhibition spécifique | % d'inhibition aspécifique |
|---|---|---|---|
| L70 (%) | 0,01 | 19 | 3 |
| | 0,1 | 96 | 6 |
| | 1 | 92 | 28 |
| L100 (%) | 0,01 | 58 | 4 |
| | 0,1 | 54 | 6 |
| | 1 | 69 | 52 |
| Huile de lupin raffiné (%) | 0,01 | 56 | 27 |
| | 0,1 | 45 | 43 |
| | 1 | 62 | 24 |
| Témoin | 0,01 | 45 | 0 |
| | 0,1 | 53 | (+2) |
| | 1 | 45 | 0 |
| Propylène | 0,01 | 0 | 2 |
| | 0,1 | 6 | 6 |
| | 1 | 13 | 7 |

4. Résultats

Ils sont exprimés comme le pourcentage d'inhibition de réparation résiduelle. La valeur 0% correspond au signal de réparation dans la condition de traitement bleu de méthylène seul.

De même, une inhibition du signal de réparation peut être parfois observée de façon aspécifique (en absence de ROS), ceci pouvant être dû à une interaction directe du composé avec l'ADN (désorption de l'ADN du puits, association aspécifique avec l'ADN qui va masquer les lésions aux enzymes de réparation...). Un contrôle consistant à incuber les agents testés avec de l'ADN prélésé est donc ajouté. Une diminution du signal dans cette condition reflète une inhibition aspécifique du composé, indépendante de ses possibles propriétés de protection de l'ADN contre des dommages oxydatifs.

Les valeurs données dans le Tableau 11 sont calculées à partir des valeurs en RLU, comme suit :

Le pourcentage d'inhibition spécifique est calculé comme la baisse relative de l'effet lésionnel dû aux oxygènes singulets générés par le bleu de méthylène (BM) éclairé, soit :

$$\frac{[\text{RLU BM}]- [\text{RLU BM + test de dilution}]}{[\text{RLU BM}]} \times 100$$

RLU : Relative Light Unit ; unité arbitraire de quantité de lumière
BM : condition bleu de méthylène + lumière

L'effet protecteur d'un composé est d'autant plus fort que son inhibition des dommages créés par l'oxygène singulet est fort, mais il est nécessaire de tenir compte pour l'interprétation de ces résultats de l'aspécificité d'inhibition du signal.

Le propylène glycol, utilisé comme contrôle interne ne présente de lui-même que de très faibles propriétés protectrices, peu significatives, vis-à-vis de l'oxygène singulet. Son effet est donc négligeable et il constitue donc le contrôle négatif.

Le composé L70 présente ainsi des propriétés protectrices certaines vis-à-vis de l'oxygène singulet. Son effet aspécifique est faible.

L'efficacité du composé L100 est également satisfaisante.

En ce qui concerne l'huile de lupin raffinée, une forte aspécificité aux trois doses testées ne permet pas de conclure avec certitude sur son efficacité.

Le témoin ne présente pas d'effet-dose, mais ne montre pas, par contre, d'aspécificité.

Enfin, aucun des produits testés n'apparaît génotoxique car aucune élévation du signal de réparation n'a été observée suite à une incubation des produits sur l'ADN.

**Revendications**

1. Utilisation de l'huile de lupin ou d'une ou plusieurs de ses fractions pour la réalisation d'un complément alimentaire anti-oxydant et/ou anti-élastase.

2. Utilisation selon la revendication 1 **caractérisée en ce que** ladite huile de lupin est obtenue à partir de farine et/ou de graines de lupin.

3. Utilisation selon la revendication 1 ou 2, **caractérisée en ce que** ladite huile de lupin est obtenue à partir d'une variété de lupin douce, en particulier à partir de lupinus albus.

4. Utilisation selon l'une des revendications 1 à 3, **caractérisée en ce que** ladite fraction est un concentrat d'huile de lupin, obtenu par distillation moléculaire de ladite huile.

5. Utilisation selon l'une des revendications 1 ou 4, **caractérisée en ce que** ladite fraction est une fraction insaponifiable contenue dans un concentrat obtenu par distillation moléculaire de ladite huile.

6. Utilisation selon la revendication 5, **caractérisée en ce que** la quantité en poids de la fraction insaponifiable dans le concentrat d'huile de lupin est de 30 % à 70 %, de préférence de 45 % à 65 %.

7. Utilisation selon l'une des revendications 1 à 6, **caractérisée en ce que** le complément alimentaire contient une fraction d'huile de lupin comprenant des dérivés phénoliques.

8. Utilisation de dérivés polyphénoliques extraits de l'huile de lupin pour la réalisation d'un complément alimentaire antioxydant et/ou antiélastase.

9. Utilisation selon l'une des revendications 7 ou 8, **caractérisée en ce que** la teneur en dérivés phénoliques est au moins égale à 20ppm.

**10.** Complément alimentaire antioxydant et/ou antiélastase comprenant de l'huile de lupin ou d'une ou plusieurs de ses fractions, **caractérisée en ce qu'**il comprend en outre de l'huile de germe de blé ou une ou plusieurs fractions de celle-ci.

**11.** Complément alimentaire selon la revendication 10, **caractérisée en ce que** ladite fraction d'huile de germe de blé est un concentrat susceptible d'être obtenu par distillation moléculaire de ladite huile.

**12.** Complément alimentaire selon la revendication 11, **caractérisée en ce que** ladite fraction d'huile de germe de blé est une fraction insaponifiable contenue dans un concentrat susceptible d'être obtenu par distillation moléculaire de ladite huile.

**13.** Complément alimentaire selon la revendication 10 ou 11, **caractérisée en ce qu'**il contient un concentrat d'huile de germe de blé en mélange avec de l'huile de lupin.

**14.** Complément alimentaire selon la revendication 13, **caractérisée en ce que** les quantités en poids de concentrat d'huile de germe de blé et d'huile de lupin varient respectivement entre 10 % et 90 %, et entre 90 % et 10%, de manière à ce que le total des quantités de ces deux huiles fasse 100 %.

**15.** Complément alimentaire selon la revendication 14, **caractérisée en ce que** les quantités en poids de concentrat d'huile de germe de blé et d'huile de lupin sont respectivement de 30 % et de 70 %.

**16.** Complément alimentaire selon l'une des revendications 10 à 15, **caractérisée en ce que** la teneur totale en poids en huile de lupin ou ses fractions et huile de germe de blé ou ses fractions dans ledit complément alimentaire est de l'ordre de 0.5 % à 10 %, de préférence de 1% à 5%.

**17.** Procédé de préparation d'un complément alimentaire selon l'une des revendications 10 à 16, **caractérisé en ce qu'**on prépare un concentrat d'huile de germe de blé par distillation moléculaire et on le mélange à de l'huile de lupin.

**Europäisches Patentamt**

**European Patent Office**

**Office européen des brevets**

**RAPPORT DE RECHERCHE EUROPEENNE**

Numéro de la demande

EP 10 15 9033

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| A | EP 0 302 769 A1 (SYNTHELABO [FR] CLINTEC NUTRITION CO [US]) 8 février 1989 (1989-02-08) * page 2, lignes 26-45; page 3, lignes 29-36; revendications 1-17 * ----- | 1-17 | INV. A23L1/30 A23L1/03 A23L1/20 |
| A | EP 0 441 672 A1 (COMMISSARIAT ENERGIE ATOMIQUE [FR]; UNIV LANGUEDOC [FR]) 14 août 1991 (1991-08-14) * colonne 1, lignes 9-28; colonne 4, lignes 10-19; exemples 1 et 2; revendications 1-13 * ----- | 1-17 | |
| A | DAVILA ET AL.: "Use of lupine as a protein-oil food source" STN CHEMICAL ABSTRACTS, vol. 8, no. 4, 31 décembre 1983 (1983-12-31), pages 23-120, XP002052109 Politecnica ISSN: 0032-3055 * abstract * ----- | 1-17 | |
| A | FR 2 692 783 A1 (EXPANCHIMIE [FR]) 31 décembre 1993 (1993-12-31) * page 1, ligne 8 - page 4, ligne 30; revendications 1-10 * ----- | 1-17 | |

| DOMAINES TECHNIQUES RECHERCHES (IPC) |
|---|
| A23L |

Le présent rapport a été établi pour toutes les revendications

| Lieu de la recherche | Date d'achèvement de la recherche | Examinateur |
|---|---|---|
| Munich | 23 juin 2010 | Georgopoulos, N |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)

**ANNEXE AU RAPPORT DE RECHERCHE EUROPEENNE
RELATIF A LA DEMANDE DE BREVET EUROPEEN NO.**

EP 10 15 9033

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

23-06-2010

| Document brevet cité au rapport de recherche | | Date de publication | Membre(s) de la famille de brevet(s) | | Date de publication |
|---|---|---|---|---|---|
| EP 0302769 | A1 | 08-02-1989 | AU | 609845 B2 | 09-05-1991 |
| | | | AU | 1972188 A | 27-01-1989 |
| | | | CA | 1324527 C | 23-11-1993 |
| | | | DE | 3863606 D1 | 14-08-1991 |
| | | | DK | 413288 A | 24-01-1989 |
| | | | FR | 2618332 A1 | 27-01-1989 |
| | | | JP | 1047721 A | 22-02-1989 |
| | | | JP | 2849653 B2 | 20-01-1999 |
| | | | LU | 90275 A9 | 14-10-1998 |
| | | | NL | 980014 I1 | 01-07-1998 |
| | | | NZ | 225523 A | 21-12-1990 |
| | | | PT | 88087 A | 30-06-1989 |
| | | | ZA | 8805368 A | 29-03-1989 |
| EP 0441672 | A1 | 14-08-1991 | FR | 2657539 A1 | 02-08-1991 |
| FR 2692783 | A1 | 31-12-1993 | AT | 175343 T | 15-01-1999 |
| | | | DE | 69322912 D1 | 18-02-1999 |
| | | | DE | 69322912 T2 | 26-08-1999 |
| | | | DK | 581624 T3 | 30-08-1999 |
| | | | EP | 0581624 A1 | 02-02-1994 |
| | | | ES | 2128400 T3 | 16-05-1999 |

EPO FORM P0460

Pour tout renseignement concernant cette annexe : voir Journal Officiel de l'Office européen des brevets, No.12/82

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- FR 9207830

- EP 441672 A

**Littérature non-brevet citée dans la description**

- Pharmacopée Européenne. 3.4.7
- *Parfumerie Cosmétique et Arôme,* 1985, 91-96

- **Keston A. S. ; Brandt R.** The fluorometric analysis of ultramicro quantities of hydrogen peroxide. *Anal. Biochem.,* 1965, vol. 11, 1-5
- *Analytical Biochemistry,* 1995, vol. 232, 37-42